# EUROPEAN PATENT SPECIFICATION

(11) **EP 2 074 969 B1**
(45) Date of publication and mention of the grant of the patent: **13.02.2013**
(21) Application number: 08254033.7
(22) Date of filing: 17.12.2008
(51) Int. Cl.: A61F 5/00

(54) **Wearable elements for implantable restriction systems**
Tragbare Elemente für implantierbare Einschränkungssysteme
Éléments portables pour systèmes de restriction implantables

(30) Priority: 18.12.2007 US 958638
(43) Date of publication of application: 01.07.2009
(73) Proprietor: Ethicon Endo-Surgery, Inc., Cincinnati, OH 45242 (US)
(72) Inventor: Marcotte, Amy L., Mason Ohio (US); Albrecht, Thomas E., Cincinnati Ohio (US); Dlugos, Daniel F., Jr., Middletown Ohio (US); Ortiz, Mark S., Milford Ohio (US)
(74) Representative: Tunstall, Christopher Stephen

(56) References cited:
- EP-A- 1 736 123
- WO-A-2007/070906
- GB-A- 2 377 157
- US-A- 4 087 864
- US-B1- 6 273 904

## Description

### FIELD OF THE INVENTION

The present invention relates to wearable elements for use with implantable restriction devices.

### BACKGROUND OF THE INVENTION

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. An example of such an adjustable gastric band is disclosed in U.S. Pat. No. 6,067,991, entitled "Mechanical Food Intake Restriction Device" which issued on May 30, 2000 It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. Traditionally, adjusting a gastric band required a scheduled clinician visit during which a hypodermic needle and syringe were used to permeate the patient's skin and add or remove fluid from the balloon. More recently, implantable pumps have been developed which enable non-invasive adjustments of the band. An external programmer communicates with the implanted pump using telemetry to control the pump. During a scheduled visit, a physician places a hand-held portion of the programmer near the gastric implant and transmits power and command signals to the implant. The implant in turn adjusts the band and transmits a response command to the programmer. During these gastric band adjustments, however, it may be difficult to determine how the adjustment is proceeding, and whether the adjustment will have the intended effect. Oftentimes, a physician may adopt a "try as you go" method based upon their prior experience, and the results of an adjustment may not be discovered until hours or days later, when the patient experiences a complete obstruction of the stomach cavity, or the band induces erosion of the stomach tissue.

Furthermore, requiring a patient to travel to a physician to have the implant powered and monitored is time consuming for both the patient and the physician. Monitoring the implant during a visit to a physician is also not necessarily the best indication of how the implant is performing when compared with data taken regularly during every day activities. Thus, there is a need for methods and devices that allow a patient to carry monitoring and powering systems. This would greatly improve the ease of use, reliability and efficiency of the implantable restriction device for both the patient and the physician. In addition, being able to monitor the conditions of the implant and of physiological conditions on a regular basis by providing devices which are wearable by the patient during daily activities may reduce the risk of missing a problem with an adjustment that could cause damage to the patient's stomach or esophageal tissue and overall health.
US2009/0306462A1 describes a system for controlling a controllable gastric band, comprising a control unit, wherein the control unit is in the form of a watch. At least one sensor is present for detecting the pressure on the gastric wall. This sensor is connected with a device for the wireless transmission of the detected pressure values to the control unit.

EP 1736123 A1 describes remote monitoring and adjustment of a food intake restriction device. The device may include a data logger with TET and telemetry coils. The data logger may be worn on a belt about the patient's waist to position the coils adjacent an injection port when the port is implanted on the patient's abdominal area.

### SUMMARY OF THE INVENTION

The present invention provides wearable elements which allow for a more comfortable and efficient way of carrying external devices related to powering and monitoring an implantable restriction device. In one exemplary embodiment, a system for forming a restriction in a patient is provided , as defined by claim 1, and includes an implantable restriction device adapted to form a restriction in a patient and an implantable communicating member configured to send and/or receive a wireless signal. The restriction device can include, for example, a gastric band and a housing in communication with the gastric band. The system further includes a wearable element configured to be worn by a patient and an external device coupled to the wearable element which is configured to send and/or receive a wireless signal to communicate with the implantable communicating member. The external device is disposable at a plurality of locations relative to the wearable element.

The external device can have a variety of configurations, and in one embodiment the external device can be an antenna positionable in proximity to the implantable communicating member and configured to receive data from the implantable communicating member. In another embodiment, the external device can be configured to send power to the implantable communicating member. In still another embodiment, the implantable communicating member can be an internal inductive coil and the external device can be an external inductive coil and the coils can be configured to resonate at substantially the same frequency to maximize power coupling. The wearable element also includes an alignment mechanism configured to indicate proper alignment between the external device and the implantable communicating member.

In another embodiment, the system can include a first external device and a second external device that are coupled to one another and are positionable at a plurality of locations on the wearable element at a distance apart from one another. In one exemplary embodiment, the first external device can be configured to receive data from the implantable communicating member and the second external device can also optionally be configured to provide power to the first external device.

The wearable element can also have a variety of configurations, and in one exemplary embodiment the wearable element can be a flexible battery adapted to flex in response to motion of a user wearing the flexible battery. In another exemplary embodiment, the wearable element can be formed from a plurality of elastic straps. In addition, the wearable element can be adjustable to a variety of patient sizes and shapes. In still another embodiment, the wearable element can be a vest, or a sash.
In other aspects, the wearable element can include at least one pocket formed therein which is movable relative to the wearable element. In one embodiment, the pocket includes at least one battery disposed therein which is configured to provide power to the external device.

Methods are also described for communicating with an implantable restriction device. In one aspect, the method can include positioning an external device on a wearable element worn by a patient at one of a plurality of locations to align the external device with an implantable communicating member on an implantable restriction device implanted in the patient. The external device can be activated to wirelessly transfer a signal through tissue to the implantable communicating member. For example, the external device can deliver energy to the implantable communicating member and/or receive data from the implantable communicating member. In addition, the external device can be an external inductive coil and the implantable communicating member can be an internal inductive coil and the method can further include using inductive coupling between the external coil and the internal coil to generate power. The two coils can be tuned to resonate at substantially the same frequency to maximize power coupling.

While the external device can be positioned at a variety of locations, in one exemplary embodiment the external device can be positioned on a skin surface in proximity to the implantable communicating member. The external device can be positioned on the wearable element or at a distance apart from the wearable element. For example, the wearable element can include a plurality of flexible straps, and the external device can be removably mated to the flexible straps in proximity to the implantable communicating member. Alternatively, or in addition, the external device can be disposed within a pocket on the wearable element. In another aspect of the invention, the wearable element can be a flexible battery. The external device can be coupled to the flexible battery and can deliver energy to the implantable communicating member generated by the flexible battery.

### BRIEF DESCRIPTION OF THE DRAWINGS

The invention will be more fully understood from the following detailed description taken in conjunction with the accompanying drawings, in which:

FIG. 1A is a representation of an implantable restriction device implanted in a patient to form a restriction in the patient's stomach;

FIG. 1B is a perspective view of the implantable restriction device of FIG. 1A;

FIG. 2A is a perspective view of one embodiment of a wearable element having pockets disposed at a plurality of locations thereon and including external devices disposed within the pockets;

FIG. 2B is a perspective view of an exemplary pocket of FIG. 2A with an external device disposed therein;

FIG. 2C is a perspective view of another embodiment of a wearable element having a plurality of pockets disposed therein;

FIG. 3A is a representation of a wearable element with an external device disposed thereon in communication with an implantable communicating member;

FIG. 3B is a representation of magnetic fields produced by the external device of FIG. 3A;

FIG. 3C is a representation of a wearable element having an array of external devices disposed thereon in communication with an implantable communicating member;

FIG. 4 is perspective view of a patient wearing another embodiment of a wearable element having an external device coupled thereto and coupled to an external device disposed at a distance apart from the external device and in proximity to the implantable restriction device of FIG. 1A;

FIG. 5A is a perspective view of yet another embodiment of a wearable element formed from flexible straps in a grid-shape and including first and second external devices which can be coupled to the wearable element at a plurality of locations;

FIG. 5B is an alternate embodiment of the wearable element of FIG. 4A having flexible straps in the form of suspenders;

FIG. 5C is an alternate embodiment of the wearable element of FIG. 4A having flexible straps in the form of a "V";

FIG. 6A is a perspective view of a double clip element coupled to an external device and adapted to attach to a wearable element;

FIG. 6B is a perspective view of a single clip element coupled to an external device and adapted to attach to a wearable element and electrically connected to a flexible battery;

FIG. 7A is a perspective view of another embodiment of a wearable element at least partially formed from a flexible battery; and

FIG. 7B is a perspective view of the wearable element of FIG. 6A having an external device coupled thereto.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices specifically described herein and illustrated in the accompanying drawings are non-liniting exemplary embodiments and that the scope of the present invention is defined solely by the claims. The features illustrated or described in connection with one exemplary embodiment may be combined with the features of other embodiments. Such modifications and variations are intended to be included within the scope of the present invention.

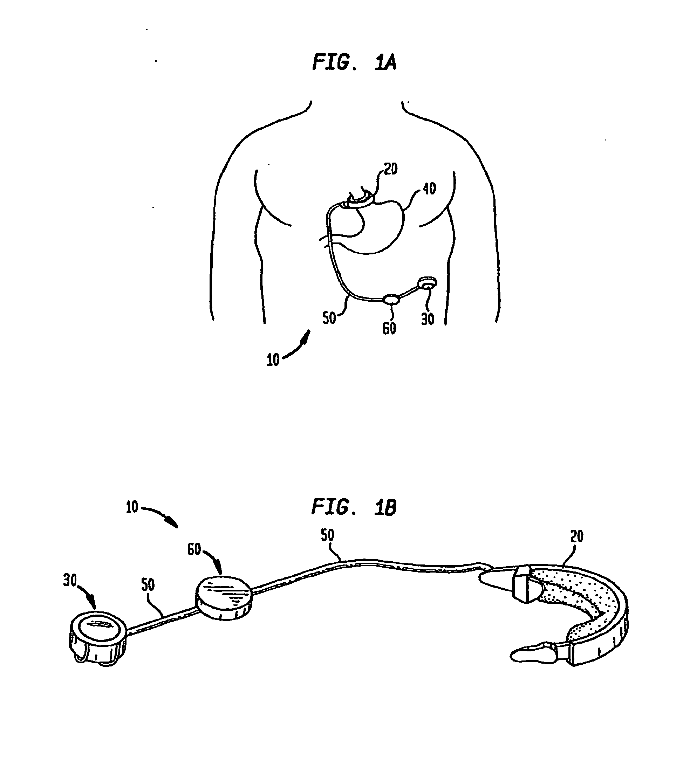

While the present invention disclosed herein can be used with a variety of restriction systems known in the art, FIGS. 1A and 1B illustrate one exemplary embodiment of a food intake restriction system 10. As shown, the system 10 generally includes an adjustable gastric band 20 that is configured to be positioned around the upper portion of a patient's stomach 40, and an injection port 30 that is coupled to the adjustable gastric band 20, e.g., via a catheter 50. The system 10 can also include an implantable communicating member 60. While the implantable communicating member 60 can have various configurations and can be positioned anywhere along the system 10, including within the injection port 30, in the illustrated embodiment, the implantable communicating member 60 is disposed within a housing positioned adjacent to the injection port 30 and is coupled to the adjustable gastric band 20. The implantable communicating member 60 can be effective to send/or receive a wireless signal. The implantable communicating member 60 can also be effective to power various components of the system, measure/monitor various conditions of the gastric band 20, and/or measure/monitor various physiological parameters. For example, the implantable communicating member 60 can be a sensor for measuring and monitoring various parameters of the system 10; an antenna such as a wire dipole antenna, a monopole antenna, or an inductive coil; and/or any other device known in the art which is capable of sending and receiving signals through tissue and/or aiding in the powering, measuring, and/or monitoring of the system 10 and/or other physiological parameters.

While not shown in FIGS. 1A and 1B, the system 10 also includes external portion that is configured to communicate via a wireless signal with the implantable communicating member 60. The external portion can have a variety of configurations, and various exemplary configurations will be discussed herein. In general, however, the external portion includes an external device which can take the form of any powering, receiving, sending, and/or monitoring device known in the art. For example, the external device can be an antenna such as a wire dipole antenna, a monopole antenna, or an inductive coil; a battery or other power supply; a monitoring device; an alignment mechanism; or any other device known in the art as needed to communicate via a wireless signal with the implantable communicating member 60. The wireless signal can take the form of any signal known in the art, for example an energy signal, a power transfer signal, and/or a data signal. In an exemplary embodiment, the external device is configured to be positioned on the skin surface above the implantable communicating member 60 (which can be implanted beneath thick tissue, e.g., over 10 cm thick) to non-invasively communicate with the implantable communicating member 60 and thereby send power to the implantable communicating member 60 and/or obtain data from the implantable communicating member 60. Alternatively, the external device can be disposed at a position remote to the implantable communicating member 60. The external portion can include a gauge or alignment mechanism that can indicate, for example, alignment between the external device and the communicating member and/or the strength of a signal being communicated between the external device and the communicating member. In addition, the gauge or alignment mechanism can provide an indication if the signal strength between the external device and the communicating member falls below an acceptable level. The indication given by the gauge or alignment mechanism can take the form of any notification means known in the art, including a light, such as an LED, an audible noise, and/or a vibration.

In one exemplary embodiment, a first external device can be configured to transmit power to the implantable communicating member 60 via a passive telemetry system. For example, a burst of radio frequency power can be sent by the first external device to the implantable communicating member which is sufficient to allow the implantable communicating member 60 to send data and measurements back to the first external device. The first external device can optionally be electrically coupled to a second external device which can record and/or display the various measurement readings or other data received by the first external device. The second external device can also send electrical power to the first external device as needed for communicating with the implantable communicating member 60. The first external device and/or the second external device can be programmed so that the first external device communicates with the implantable communicating member 60 at random intervals or at predetermined time periods so that the patient is not required to activate any device in order for the first external device and the implantable communicating member 60 to communicate. Alternatively, communication between the first external device and the implantable communicating member 60, or between the first external device and the second external device, can be manually activated by the patient or other user.

In one exemplary embodiment, the second external device is a rechargeable battery system configured to send power to the first external device as needed. The second external device can also include circuitry configured to control the amount of power sent through the first external device to the implantable communicating member 60. Alternatively, a single external device can perform all, any one, or any combination of the functions noted above. In still another embodiment, the external device can include multiple battery systems in connection with one another to maximize available power as will be described in more detail below. A person skilled in the art will appreciate that any number of external devices can be used to perform any number of functions needed to power, monitor, and otherwise wirelessly communicate with the implantable communicating member 60. In the same way, a single external device can be configured to perform all of the above noted functions as needed.

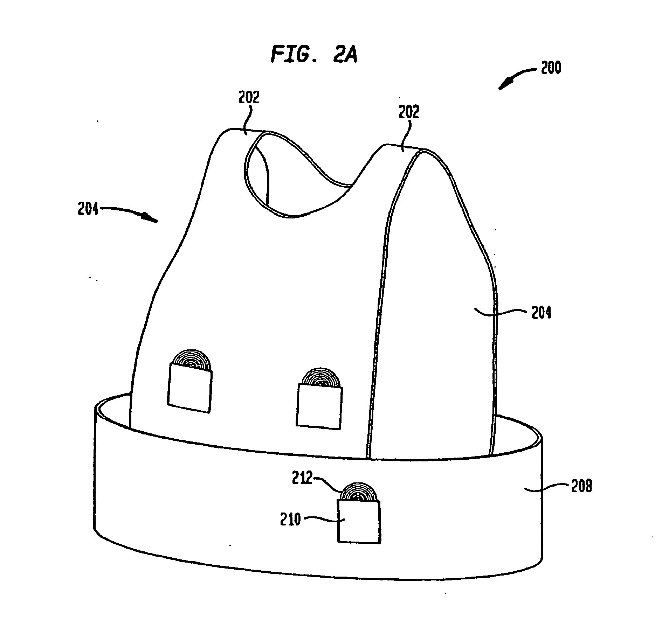

The external portion also includes a wearable element that is configured to be worn by a patient, and that is coupled to one or more external devices. The wearable element can be particularly effective to allow the external device(s) to be worn by the user, thus allowing for communication between the external device(s) and the implantable communicating member as may be desired. FIGS. 2A - 6B illustrate various exemplary wearable elements. In each of the various embodiments, external devices are integral with or coupled to a wearable element which allows for a more comfortable and efficient way of carrying the external devices.

As used herein, the term "wearable" refers to an article of manufacture designed to be worn on or borne by the body or a portion of the body of a wearer. When in the form of a garment, the wearable element can be, for example, in the form of a vest, a shirt, a tank top, a bodysuit, a jacket. The term "wearable" can encompass not only garments, but also a sash.

In use, the wearable element can generally be wearable over some portion of a patient's body. The wearable element can be configured to be fitted tightly to the portion of the patient's body so as to fit under a patient's clothing. It can also be configured to fit loosely over a patient's body or over a patient's clothing. Adjustability elements can be included on the wearable element so that the wearable element can be adapted to fit patients of various sizes and shapes. The wearable element can be made of various materials, but a light and resilient material is preferred to optimize comfort and ease of use for the patient. In an exemplary embodiment, the wearable element is made from materials commonly used in fabric and textile applications. For example, the wearable element can be made from elastomeric yarns (such as spandex) and/or comfort yarns (such as nylon, polyester, or cotton). Any combination of materials can be formed as necessary to maximize comfort and ease of use for the patient, as well as to provide ease of cleaning. The wearable element can also be adapted to be worn for extended lengths of time by the patient or it can be adapted to be worn only at specific times as needed for powering, monitoring, and/or otherwise communicating with the implantable communicating member.

In one exemplary embodiment shown in FIG. 2A, a wearable element is provided in the form of a vest 200. The vest 200 is made to fit over and around a patient's upper torso, and thus generally includes shoulder straps 202 that rest on the patient's shoulders. The vest 200 can also include arm cut-outs 204 in each side through which a patient's arms can fit. In the illustrated embodiment, the vest 200 also includes a belt or band 208 configured to surround the lower torso or waist of the patient and which can be tightened or loosened as needed for a better fit to the patient. Alternatively or in addition, adjustability elements can be provided on the vest 200 so that it can be adjusted to fit patients of various sizes and shapes. The adjustability elements can be in the form of buckles, clasps, buttons, snaps, ties, straps, zippers, VelcroTM, etc.

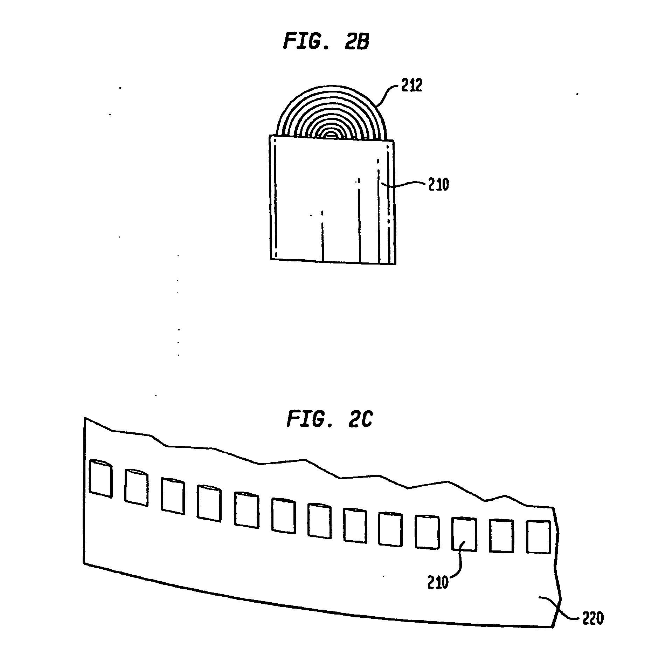

As also shown in FIG 2A, at least one pocket 210 can be formed in or disposed on an exterior surface of the vest 200. As shown in FIGS. 2A and 2B, the pocket 210 can be formed to a size large enough to contain at least one external device 212, such as an inductance coil, an antenna, a battery, or other circuitry, as described in detail above. The pocket 210 can be removably attached to the vest 200 via Velcro™, stitching, adhesive, or any other attachment means known in the art, so that the pocket 210 can be placed in proximity to an implantable communicating member implanted within the patient or anywhere else on or in the wearable element as needed. A removable pocket 210 allows for adjustments to be made to the location of the external device 212 so as to properly align the external device 212 with the implantable communicating member. As an example, in FIG. 2A, the pocket 210 is shown in three alternate locations on the vest 200, and it will be understood by those skilled in the art that the pocket 210 can be attached at any location on the vest 200 as needed. The pocket 210 can also be integrally formed in the vest 200 at a fixed location, and the vest 200 can include any number of pockets 210.

In another exemplary embodiment shown in FIG. 2C, a wearable element 220 is provided having a plurality of pockets 210 formed therein or disposed thereon. This allows for multiple external devices 212 to be placed in pockets 210 and carried by the patient as needed. Providing a plurality of pockets 210 on the wearable element 220 allows for greater control in weight distribution of multiple external devices, as well as flexibility in placement for patient comfort.

In use, for example, one pocket attached to a wearable element can be placed in proximity to an implantable communicating member and can hold an external device, such as an antenna, for communicating with the implantable communicating member. Simultaneously, another pocket can be disposed towards the bottom of the wearable element and can hold another external device, such as a rechargeable battery system or reader device, that is connected to the antenna.

As another example, two pockets can be disposed next to each other on a wearable element in proximity to the implantable communicating member, each holding an external device. A first external device can be an antenna for obtaining data or measurements from the implantable communicating member and a second external device can be an inductive coil for providing power to the implantable communicating member. Alternatively or in addition, one pocket can hold an alignment mechanism for monitoring proper alignment of the first and/or second external devices with the implantable communicating member.

In a final example most clearly illustrated in FIG. 2C, multiple pockets 210 can be disposed in different locations on the wearable element 220 to hold multiple battery units. If it is determined that a battery should be used to provide power to a certain external device, then having just one battery unit for all the powering needs of the implant could be extremely heavy for the patient to carry in a single pocket on a wearable element. Thus, several smaller battery units can be connected to one another and used in place of the single battery. Evenly spacing multiple pockets 210 containing the smaller battery units around the wearable element 220 could prevent the discomfort of having a single heavy battery unit attached to one location on the patient. The smaller battery units can be disposed in multiple pockets around the wearable element 220 so that the weight is distributed in a more even fashion.

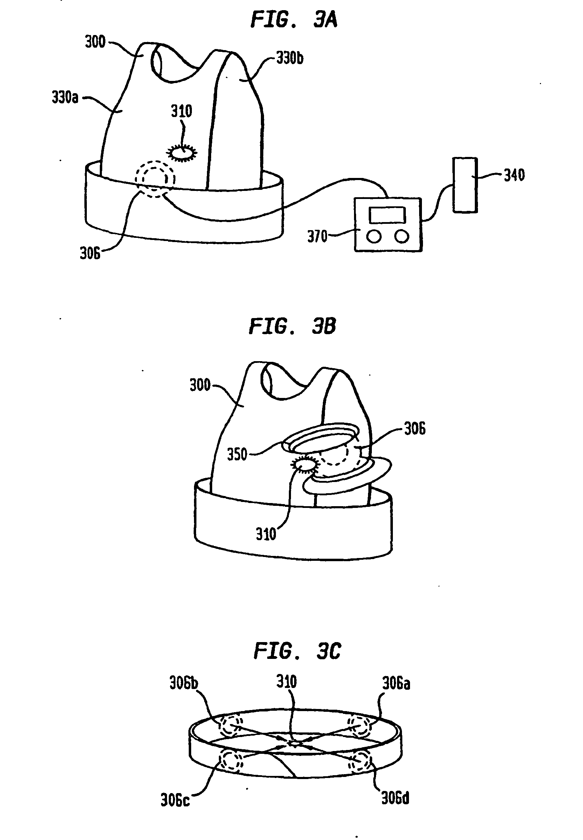

FIGS. 3A and 3B illustrate an embodiment of a wearable element in the form of a vest 300 having an external device in the form of an external inductive coil 306 adapted to communicate through inductive coupling with an implantable communicating member in the form of an internal inductive coil 310. FIG. 3B illustrates magnetic fields 350 produced by the external inductive coil 306 that are capable of generating current within the internal inductive coil 310 through methods well known in the art. The internal and external inductive coils 306, 310 can be configured to resonate at substantially the same frequency to maximize power coupling. The vest 300 can fit over and/or around the torso of a patient and can contain adjustability elements for fitting the vest 300 to patients of various sizes and shapes. In addition, the vest 300 can include an external device in the form of a reader device 370 for powering the external coil 306 and/or for receiving and recording data from an implantable communicating member.

The vest 300 can generally be wearable over a patient's torso and can be configured to be fitted tightly to the torso so as to fit under a patient's clothing or to fit loosely over a patient's body or over a patient's clothing. The external inductive coil 306 can be formed from wound metal wire, for example copper wire, and can conform to various sizes and shapes as needed for powering the internal inductive coil 310, as is well known in the art. The external inductive coil 306 can be adapted to be positioned adjacent to a tissue surface and in proximity to the internal inductive coil 310. For example, the external inductive coil 306 can be formed in a front panel 330a or a rear panel 330b of the vest 300. The external inductive coil 306 can be formed integrally with the vest 300 or can be disposed on an exterior or interior surface of the vest 300 and can be attached to or disposed within the vest 300 in any way known in the art which is effective to allow proper alignment of the external inductive coil 306 with the internal inductive coil 310. Thus, the external inductive coil 306 can optionally be removably attached to the vest 300 so that the external coil 306 can be moved and aligned as needed. Alternatively, the external inductive coil 306 can be integrally formed in an interior portion of the vest 300 with a predetermined alignment based on where the internal inductive coil 310 will be positioned. The internal coil 310 can also be formed of metal wire, for example copper magnet wire, and can be of a size and shape to achieve the desired power and/or signal coupling from the corresponding external inductive coil 306 or inductive coils 306a- 306d.

In an exemplary embodiment, the external inductive coil 306 can be in electrical communication with the external reader 370. The external reader 370 can provide power to the external inductive coil 306 taken from any power source 340 known in the art, for example a battery or wall electrical outlet. The external reader 370 can be configured so that a user can control the amount of power coupled through the external inductive coil 306 to the internal inductive coil 310, as well as other aspects of using the external inductive coil 306. The external reader 370 can be configured in various ways. For example, it can be positioned on the vest 300, in a hand-held device, or at a position remote from the vest 300, as shown in FIG. 3A. As will be appreciated by those skilled in the art, the external inductive coil 306 can alternatively be directly coupled to a power source 340. The external reader 370 can optionally be configured to receive, record, and/or display data received from a communicating member. The data can be received by any methods known in the art. For example, the external inductive coil 306 can include an antenna configured for receiving data from a communicating member and/or the external reader 370 can include an antenna for receiving the data from a communicating member. The data and measurements taken from the communicating member can be displayed or reworded by the external reader 370 for later review.

In an shown in FIG. 3C, a wearable element is shown in the form of a sash or belt 360 having an array of four external coils 306a, 306b, 306c, 306d disposed around the belt 360 so as to surround an implanted interior coil 310. This configuration allows inductive coupling to occur with the interior coil 310 in two perpendicular directions. A person skilled in the art will appreciate that any number of coils can be disposed around or over any wearable element to inductively couple with an implanted coil or any number of implanted coils.

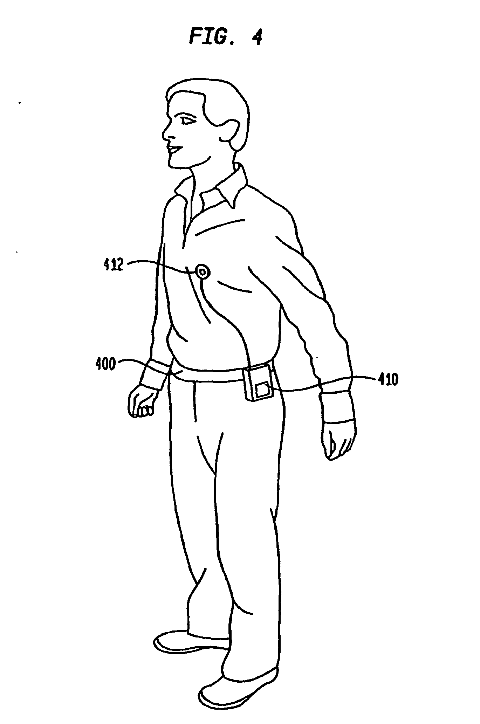

FIG. 4 shows a wearable element in the form of a belt, sash, or band 400. The band 400 is configured to be disposed around the waist, stomach, or hips of a patient and to hold a first external device 410 so that a patient is prevented from having to wear a bulky device under their clothing. The band 400 can also include adjustability elements such as a buckle, button, clasp, zipper, VelcroTM, or string laces, to adjust the size of the band 400 to fit patients of various sizes and shapes. In one embodiment, as shown, the first external device 410 can be a processor or reader device, and it can be electrically connected to a second external device, such as an antenna 412, which can be placed under a patient's clothes in proximity to an implantable communicating member. An adhesive or other attachment mechanism can be used to maintain the antenna in a fixed position. In this way, the antenna 412 can send power to and/or receive data from the implantable communicating member and send the data to the second external device 410 which can display or record the data. Alternatively or in addition, the second external device 410 can be in the form of a battery unit or other powering device for providing power to the antenna 412. In addition, the band 400 can include an alignment mechanism, as described above, which monitors the alignment between the antenna 412 and the implantable communicating member. The alignment mechanism can indicate, for example, whether alignment between the antenna 412 and the communicating member should be readjusted and/or realigned for better communication. The indication given by the alignment mechanism can take the form of any notification means known in the art, including a light, such as an LED, an audible noise, and/or a vibration.

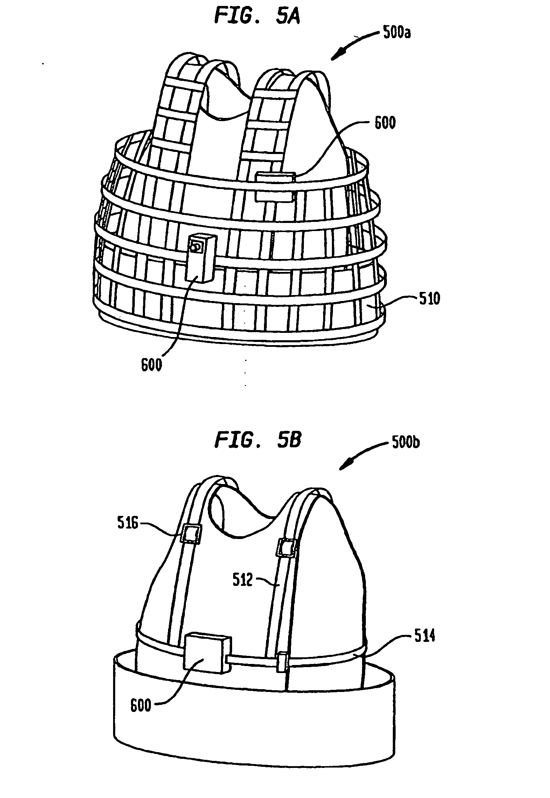

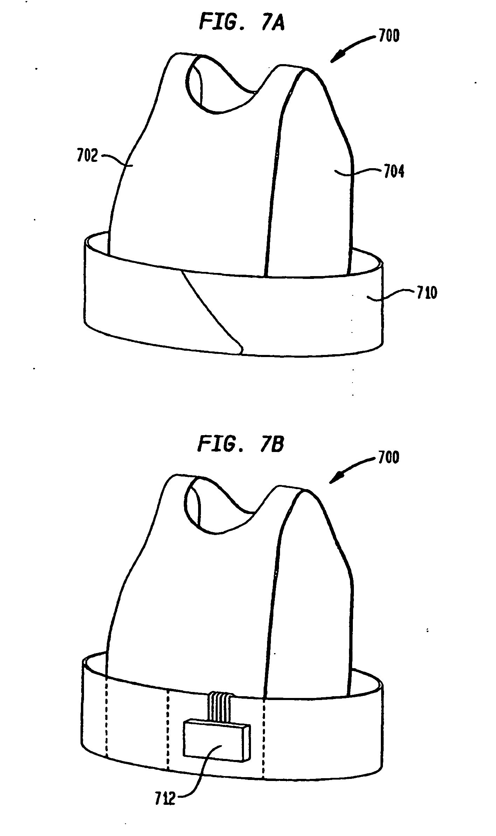

In another embodiment, FIGS. 5A-5C illustrate wearable elements 500a, 500b, and 500c formed from straps which are worn on the upper torso of a patient. The wearable elements 500a, 500b, and 500c can be placed over a vest as described above in reference to FIG. 2A, or over a wearable element made from a flexible battery, as described in detail below in reference to FIGS. 7A and 7B. Alternatively, the wearable elements 500a, 500b, and 500c can be worn over or under a patient's clothing.

In the embodiment illustrated in FIG. 5A, the straps are elastic so as to fit tightly over a vest or a patient's torso and are in the form of checkered or grid-shaped straps 510. The wearable element 500a can include adjustability elements, for example a buckle, button, clasp, or zipper, to adjust the size to fit under or over a patient's clothing and to fit to the specific size and shape of a patient. The grid-shaped straps 510 can be made from various materials, such as a non-elastic material.

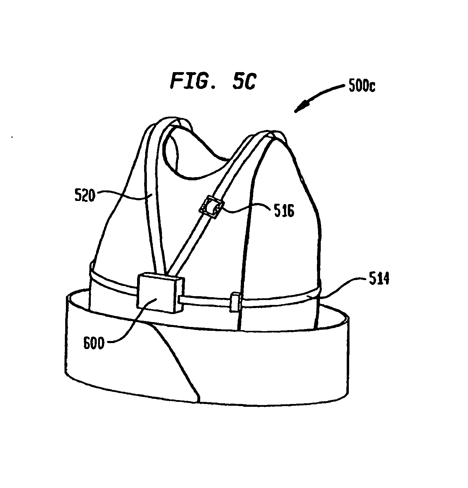

FIGS. 5B and 5C show alternate strap patterns or shapes for the wearable elements 500b and 500c, respectively. In FIG. 5B, the straps are in the shape of suspenders 512 connected to a horizontal strap 514, and in FIG. 5C the straps are in the shape of a "V" 520, also connected to a horizontal strap 514. The straps 512, 520 and/or the horizontal straps 514 can include a buckle 516 or other clasp mechanism to facilitate size adjustments. A person skilled in the art will appreciate that strap shapes and arrangements are in no way limited to these three embodiments and can take the form of any shape or arrangement as needed.

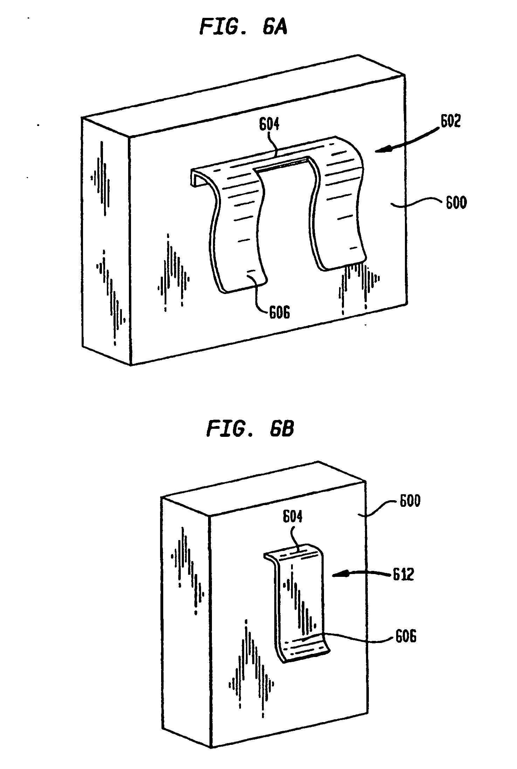

In an exemplary embodiment, in use, each of the wearable elements of FIGS. 5A-5C is adapted to couple to an external device. While various mating techniques can be used to mate an external device to the straps, FIGS. 6A and 6B illustrate various clips for engaging the straps. In particular, clips 602, 612 are particularly advantageous as they allow an external device 600 to be clipped to various locations on the wearable elements 500a, 500b, and 500c described above. The clips 602, 612 can be made of various materials such as plastic, metal, etc. and each have a horizontal portion 604. Clip 612 has one leg 606 extending perpendicularly from the horizontal portion 604, and clip 602 has two legs 606 extending from the horizontal portion 604. The horizontal portion 604 can be rigidly attached to the external device 600 by any mating means known in the art, such as rivets, screws, glue, etc. The legs 606 can be rigid or flexible, but in the illustrated embodiment they are flexible and have a curvature associated with their shape which is effective to cause at least a portion of the legs 606 to press against the external device 600. In use, the legs 606 can be flexed away from the device 600 to engage the straps of the wearable elements in FIGS. 5A-5C. After the straps are engaged, the legs 606 can be released and will flex back to their original position pressed against the external device 600.

FIG. 7A shows another embodiment of a wearable element. In certain embodiments, it may be necessary to provide power to an external device. Providing this power through a rechargeable battery would be the most convenient for the patient, although conventional batteries, such as lead-acid batteries are extremely heavy. Thus,

FIG. 7A shows a wearable element in the form of a flexible battery vest 700 made, at least in part, from a thin flexible battery. The flexible battery can be, for example a lithium polymer battery such as Solicore's Flexion™ line of lithium polymer batteries or an ultra-thin organic radical battery from, for example, NEC®, both of which are thin and flexible and can be made into the shape of any article of clothing as needed. In addition, both the lithium polymer battery and the organic radical battery can be charged and recharged quickly and have a low weight. These characteristics provide optimum batteries from which to form a wearable article of clothing, such as the flexible battery vest 700, that a patient can wear to provide power to an external device and that flexes in response to the motion of a patient. The shape of the wearable flexible battery can vary. In the illustrated embodiment, the wearable element includes a from panel 702 and a rear panel 704, each of which can be made from a flexible battery, and attached to or integrally formed with one another to form the flexible battery vest 700. In the illustrated embodiment, the flexible battery vest 600 also includes a belt or band 710 which can also be made from a flexible battery if desired. Both the flexible battery vest 700 and the band 710 can include adjustability elements to adjust the size to fit patients of various sizes and shapes.

In another embodiment, the wearable element can be in the form of an adhesive patch that can be applied to one of the other embodiments of wearable elements described herein. The adhesive patch can be formed from a flexible battery, and it can be attached directly to a wearable element or disposed within a pocket that is attached to a wearable element, as described above in preference to FIGS. 2A-2C. A person skilled in the art will appreciate that any wearable element configured to be worn on any part of the body can be formed of a flexible battery with the same effects and characteristics described herein.

As further shown in FIG. 7B, the flexible battery vest 700 can also include an external device 712 that is electrically coupled to the flexible battery vest 700 and can be placed under the clothing of a patient or attached to the flexible battery vest 700. Alternatively, the wearable elements 500a, 500b, and 500c of FIGS. 5A-5C can be placed over the flexible battery vest 700 and adjusted to fit tightly thereto or under the flexible battery vest 700 to fit tightly to the patient. An external device can be clipped to the wearable elements 500a, 500b, and 500c and can be in electrical communication with the flexible battery vest 700, thereby providing power as needed to the external device.

In use, in each of the various embodiments disclosed herein, the external device can be positioned on a wearable element worn by a patient at one of a plurality of locations to align the external device with an implantable communicating member on an implantable restriction device implanted in a patient. Once positioned as desired, the external device can be activated to transfer a signal through tissue to the implantable communicating member.

In one exemplary embodiment, the wearable element can be placed on or around the patient depending on the form of the wearable element. Once placed on or around the patient, adjustability elements can be adjusted to fit the wearable element to the patient, whether under the patient's clothing or over the patient's clothing. Once a proper and comfortable fit is attained, external devices can be attached to the wearable element as necessary and as will be described in detail below.

In the embodiment shown in FIG. 2A, the vest 200 can be placed over a patient's head to rest on a patient's shoulders. Alternatively, the belt or band 208 can be opened to allow the vest 200 to be slipped sideways over a patient's body. Once the vest 200 is situated on a patient's torso, the adjustability elements can be modified to properly fit the vest 200 to the patient. When the vest 200 is fitted to the patient comfortably, one or more pockets containing one or more external devices can be removably attached to the vest 200 and aligned as needed. For example, a pocket 210 containing a external device 212 can be placed in proximity to and in alignment with an implantable communicating member to send power to and/or receive data from the implantable communicating member. A person skilled in the art will appreciate that any number of pockets containing external devices can be removably positioned on or about the vest 200 as needed. Alternatively, the vest 200 can be prepared with one or more pockets attached in predetermined locations to correspond to a known implant location or other requirement. In this case, an external device 212 can be placed into the pocket 210 once the vest 200 is fitted to the patient.

Once the external devices 212 are in the proper location with the proper alignment, if necessary they can be activated by a physician, a user, or by self-actuation to perform any number of actions. For example, where the external device is an antenna, activation of the antenna can be effective to send power or energy to an implantable communicating member to enable the implantable communicating member to send back data to the antenna regarding the data and other measurements taken concerning the implantable restriction device. Alternatively, where the external device is an inductive coil, activation of the coil can be effective to communicate with an implantable communicating member, for example an inductive coil, to thereby transfer power to the implantable restriction system through inductive coupling.

In the embodiment shown in FIGS. 3A-3C, the vest 300 and the belt 360 can be placed over and/or around the patient and the size adjusted to properly fit the patient. The external inductive coil 306 can be activated to inductively couple with the internal inductive coil 310 so that electricity is generated within the internal inductive coil 310. In an exemplary embodiment, the external reader 370 and/or the external inductive coil 306 can send and/or receive data and/or measurements from an implantable communicating member as needed.

In the aspect shown in FIG. 4, the belt or band 400 can be placed around the waist or stomach area of a patient and the size adjusted to properly fit the patient. The external device 410 can be attached to the band 400 by a buckle, clip, or other attachment means so that the patient does not have to wear a bulky external device under clothing. Where the external device is an antenna 412, it can be placed on the patient's skin in proximity to an implantable communicating member to communicate with the implantable communicating member as needed. Once the antenna 412 is properly aligned within the implantable communicating member, the antenna 412 can be placed in electrical communication with the external device 410 and can thereby receive power that can be transmitted to the implantable communicating member and send data received from the implantable communicating member to the external device 410. The external device 410 can then record the data obtained from the implantable communicating member and display the data as a read-out, or save the data for later review by a physician.

In the embodiment shown in FIGS. 5A-5C, the wearable elements 500a, 500b, and 500c can be placed under a patient's clothing, over a patient's clothing, or over one of the wearable elements described above and adjusted to fit the patient. One or more external devices can then be clipped to the flexible straps using the single or double clips shown in FIGS. 6A and 6B. The external devices can be activated to send power to the implantable communicating member, receive data from the implantable communicating member, or simply monitor the implantable restriction system at various predetermined or random intervals.

In the embodiment shown in FIGS. 7A and 7B, the flexible battery vest 700 is placed over and around a patient's torso and secured thereto. The battery can be activated so as to provide power as needed to any external devices attached to or disposed over or under the flexible battery vest 700.

One skilled in the art will appreciate further features and advantages of the invention based on the above-described embodiments. Accordingly, the invention is not to be limited by what has been particularly shown and described, except as indicated by the appended claims.

## Claims

1. A system for forming a restriction in a patient, comprising:
an implantable restriction device (10) adapted to form a restriction in a patient, the implantable restriction device including an implantable communicating member (60) configured to send and/or receive a wireless signal: and
a wearable element (200) configured to be worn by a patient, and having an external device (212) coupled thereto and configured to send and/or receive a wireless signal to communicate with the implantable communicating member, wherein the external device is disposable at a plurality of locations on the wearable element;
**characterised in that** the wearable element is in the form of a vest, a sash, a shirt,
a tank top, a bodysuit or a jacket and the wearable element includes an alignment mechanism configured to indicate proper alignment between the external device and the implantable communicating member.

2. The system of claim 1, wherein the external device comprises an antenna positionable in proximity to the implantable communicating member and configured to receive data from the implantable communicating member.

3. The system of any preceding claim, wherein the external device is configured to send power to the implantable communicating member.

4. The system of any preceding claim, wherein the external device comprises an exterior inductive coil and the implantable communicating member comprises an internal inductive coil.

5. The system of claim 4, wherein the external inductive coil is configured to resonate at substantially the same frequency as the internal inductive coil to maximize power coupling.

6. The system of any preceding claim, wherein the external device comprises a first external device and the system includes a second external device, wherein the first and second external devices are coupled to one another and positionable at a plurality of locations on the wearable element at a distance apart from one another.

7. The system of claim 8, wherein the first external device is configured to receive data from the implantable communicating member and the second external device is configured to store data received by the first external device.

8. The system of claim 6 or 7, wherein the second external device is configured to provide power to the first external device.

9. The system of any preceding claim, wherein the wearable element comprises a flexible battery adapted to flex in response to motion of a user wearing the flexible battery.

10. The system of any preceding claim, wherein the wearable element is formed from a plurality of elastic straps.

11. The system of any preceding claim, wherein the wearable element is adjustable to a variety ofpatient sizes and shapes.

## Patentansprüche

1. System zur Bildung einer Einschränkung in einem Patienten, das Folgendes umfasst:
eine implantierbare Einschränkungsvorrichtung (10) zur Bildung einer Einschränkung in einem Patienten, wobei die implantierbare Einschränkungseinrichtung ein implantierbares Kommunikationselement (60) aufweist,
das zum Senden und/oder Empfangen eines drahtlosen Signals konfiguriert ist; und
ein tragbares Element (200), das so konfiguriert ist,
dass es von einem Patienten getragen wird und eine damit verbundene externe Vorrichtung (212) aufweist,
die zum Senden und/oder Empfangen eines drahtlosen Signals zur Kommunikation mit dem implantierbaren Kommunikationselement konfiguriert ist, worin die externe Vorrichtung an einer Vielzahl von Stellen auf dem tragbaren Element angeordnet werden kann;
**dadurch gekennzeichnet, dass** das tragbare Element in Form einer Weste, einer Schärpe, eines Hemds, eines Trägerhemds, eines Bodysuits oder einer Jacke vorliegt und das tragbare Element einen Ausrichtungsmechanismus aufweist, der so konfiguriert ist, dass er die richtige Ausrichtung zwischen der externen Vorrichtung und dem implantierbaren Kommunikationselement anzeigt.

2. System nach Anspruch 1, worin die externe Vorrichtung eine Antenne umfasst, die in der Nähe des implantierbaren Kommunikationselements angeordnet werden kann und zum Empfangen von Daten von dem implantierbaren Kommunikationselement konfiguriert ist.

3. System nach einem der vorhergehenden Ansprüche, worin die externe Vorrichtung zum Aussenden von Energie an das implantierbare Kommunikationselement konfiguriert ist.

4. System nach einem der vorhergehenden Ansprüche, worin die externe Vorrichtung eine externe Induktionsspule umfasst und das implantierbare Kommunikationselement eine interne Induktionsspule umfasst.

5. System nach Anspruch 4, worin die externe Induktionsspule so konfiguriert ist, dass sie zur Maximierung der Energiekopplung im Wesentlichen mit derselben Frequenz schwingt wie die interne Induktionsspule.

6. System nach einem der vorhergehenden Ansprüche, worin die externe Vorrichtung eine erste externe Vorrichtung umfasst und das System eine zweite externe Vorrichtung umfasst, worin die ersten und zweiten externen Vorrichtungen miteinander verbunden sind und an einer Vielzahl von Stellen auf dem tragbaren Element im Abstand voneinander angeordnet werden können.

7. System nach Anspruch 8, worin die erste externe Vorrichtung zum Empfangen von Daten von dem implantierbaren Kommunikationselement konfiguriert ist und die zweite externe Vorrichtung zum Speichern von Daten, die von der ersten externen Vorrichtung empfangen wurden, konfiguriert ist.

8. System nach Anspruch 6 oder 7, worin die zweite externe Vorrichtung so konfiguriert ist, dass sie die erste externe Vorrichtung mit Energie versorgt.

9. System nach einem der vorhergehenden Ansprüche, worin das tragbare Element eine flexible Batterie umfasst, die sich als Reaktion auf eine Bewegung eines Anwenders, der die flexible Batterie trägt, biegen kann.

10. System nach einem der vorhergehenden Ansprüche, worin das tragbare Element aus einer Vielzahl von elastischen Gurten geformt ist.

11. System nach einem der vorhergehenden Ansprüche, worin das tragbare Element an eine Vielzahl von Größen und Formen von Patienten angepasst werden kann.

## Revendications

1. Système destiné à former une restriction dans un patient, comportant :
un dispositif (10) de restriction implantable conçu pour former une restriction dans un patient, le dispositif de restriction implantable comprenant un organe (60) de communication implantable configuré pour envoyer et/ou recevoir un signal sans fil ; et
un élément (200) portable configuré pour être porté par un patient, et doté d'un dispositif (212) externe accouplé à ce dernier et qui est configuré pour envoyer et/ou recevoir un signal sans fil afin de communiquer avec l'organe de communication implantable, le dispositif externe pouvant être disposé au niveau d'une pluralité d'emplacements sur l'élément portable ;
**caractérisé en ce que** l'élément portable se présente sous la forme d'un débardeur, une écharpe, une chemise, un pull-over sans manches, une combinaison ou une veste et l'élément portable comprend un mécanisme d'alignement configuré pour indiquer l'alignement correct entre le dispositif externe et l'organe de communication implantable.

2. Système selon la revendication 1, dans lequel le dispositif externe comporte une antenne positionnable à proximité de l'organe de communication implantable et qui est configurée pour recevoir des données de l'organe de communication implantable.

3. Système selon l'une quelconque des revendications précédentes, dans lequel le dispositif externe est configuré pour envoyer de la puissance à l'organe de communication implantable.

4. Système selon l'une quelconque des revendications précédentes, dans lequel le dispositif externe comporte une bobine d'induction extérieure et l'organe de communication implantable comporte une bobine d'induction intérieure.

5. Système selon la revendication 4, dans lequel la bobine d'induction externe est configurée pour résonner à sensiblement la même fréquence que la bobine d'induction intérieure pour optimiser le couplage de puissance.

6. Système selon l'une quelconque des revendications précédentes, dans lequel le dispositif externe comporte un premier dispositif externe et le système comprend un second dispositif externe, les premier et second dispositifs externes étant accouplés l'un à l'autre et positionnables à une pluralité d'emplacements sur l'élément portable à une certaine distance l'un de l'autre.

7. Système selon la revendication 8, dans lequel le premier dispositif externe est configuré pour recevoir des données de l'organe de communication implantable et le second dispositif externe est configuré pour mémoriser les données reçues par le premier dispositif externe.

8. Système selon la revendication 6 ou 7, dans lequel le second dispositif externe est configuré pour fournir de la puissance au premier dispositif externe.

9. Système selon l'une quelconque des revendications précédentes, dans lequel l'élément portable comporte une batterie flexible conçue pour fléchir en réponse au mouvement d'un utilisateur qui porte la batterie flexible.

10. Système selon l'une quelconque des revendications précédentes, dans lequel l'élément portable est formé à partir d'une pluralité de bandes élastiques.

11. Système selon l'une quelconque des revendications précédentes, dans lequel l'élément portable est ajustable pour correspondre à différentes tailles et formes de patient.
